(19) Europäisches Patentamt
European Patent Office
Office européen des brevets

(11) Publication number: 0 082 732 A2

# (12) EUROPEAN PATENT APPLICATION

(21) Application number: 82306934.9

(22) Date of filing: 23.12.82

(51) Int. Cl.[3]: G 03 C 7/32
C 07 D 487/04, C 07 D 271/04
C 07 D 401/06
//(C07D487/04, 249/00, 231/00),
(C07D487/04, 235/00, 231/00),
(C07D271/04, 231/00, 221/00),
(C07D401/06, 231/00, 215/00)

(30) Priority: 23.12.81 JP 213765/81

(43) Date of publication of application:
29.06.83 Bulletin 83/26

(84) Designated Contracting States:
DE FR GB

(71) Applicant: KONISHIROKU PHOTO INDUSTRY CO. LTD.
No. 26-2, Nishishinjuku 1-chome Shinjuku-ku
Tokyo 160(JP)

(72) Inventor: Uemura, Morito
144, 5-2-1, Hinodai
Hino-shi Tokyo(JP)

(72) Inventor: Sugita, Hiroshi
5-26 Sanda-machi
Hachioji-shi Tokyo(JP)

(72) Inventor: Masuda, Kosaku
5-16-2-106 Tamagawa-cho
Akishima-shi Tokyo(JP)

(74) Representative: Ellis-Jones, Patrick George Armine et al,
J.A. KEMP & CO. 14 South Square Gray's Inn
London WC1R 5EU(GB)

## (54) A blocked magenta dye forming coupler.

(57) A blocked magenta dye forming coupler is disclosed having the formula

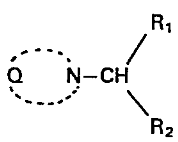

wherein Q represents a group of non-metallic atoms necessary to form a magenta dye forming coupler together with a nitrogen atom; and

$R_1-CH-R_2$
|

represents a blocking radical which, after reacting with an oxidized developing agent, is released from said blocked magenta dye forming coupler where $R_1$ represents Z-NHCO-, Z-CO- or Z-O-CO- wherein Z is a 5- or 6-membered heterocyclic ring, which may have a condensed ring; and $R_2$ represents X-NHCO-, X-CO-, X-O-CO, NC-, $O_2N$-, HOOC- (wherein X is an alkyl or an aryl radical) or a radical as defined in $R_1$.

EP 0 082 732 A2

# A BLOCKED MAGENTA DYE FORMING COUPLER

The present invention relates to a novel blocked silver halide photographic magenta dye forming coupler, and more particularly to a blocked photographic magenta dye forming coupler which is not only capable of color-forming at a high rate and of producing a high maximum color density but also improved to be so stable that the color formability thereof is not deteriorated with time.

As conventionally known magenta dye forming couplers there are pyrazolone-triazole, pyrazolobenzimidazole, indazolone and pyrazolone couplers. There are also known two-equivalent type magenta dye forming couplers wherein a silver-usage efficiency is enhanced by introducing a substituent into the active site of these magenta dye forming couplers.

These conventionally known two-equivalent type magenta dye forming couplers include those couplers of, e.g., the halogen-substituted type (U.S. Patent No. 3,006,579), the aryloxy-substituted type (U.S. Patent No. 3,419,391), the carbonyloxy-substituted type (U.S. Patent Nos. 3,311,476 and 3,422,521, Japanese Patent Publication Open to Public Inspection (hereinafter referred to as Japanese patent O.P.I. Publication) No. 129535/1974, etc.), the nitrogen or sulfur-substituted type (Japanese Patent O.P.I. Publication Nos. 53435/1974, 53436/1974,

53372/1975, 122935/1975, etc.), the carbon-substituted type (U.S. Patent No. 2,632,702, Japanese Patent O.P.I. Publication No. 37646/1976, etc.), the substituted methylene-substituted type (British Patent No. 963,461, Japanese Patent Examined Publication No. 4036/1959, etc.), the methylene, alkylidene or arylidene-bis type (U.S. Patent No. 2,618,641, British Patent Nos. 736,859 and 968,461, Japanese Patent Examined Publication Nos. 16110/1969, 26589/1969 and 37854/1974, Japanese Patent O.P.I. Publication No. 29638/1974), and the like.

These two-equivalent type magenta dye forming couplers have such characteristics that both the dye forming rate thereof and the developed color's maximum density thereof are higher than those of 4-equivalent type couplers, and the like, but the characteristics are still not necessarily sufficient. In addition, the foregoing known two-equivalent type couplers, since they are active in themselves, have the disadvantage that they are unstable. Therefore, they have the problems that not only do they tend to produce fog and yellow stain in the non-color forming area as well as in the low density area during the color development thereof but also the color formability thereof becomes deteriorated with time. In recent years, the color photographic technology has been aiming at both higher photographic speed and more highly satisfactory graininess.

The use of a large amount of a silver halide for the purpose of obtaining a high sensitivity supplies excessively the

oxidized product of a developing agent produced therefrom (i.e., oxidized aromatic primary amine developing agent) and as a result produces a large amount of a dye, resulting in the deterioration of the graininess. In order to improve this, development inhibitor releasing compounds, competing couplers or the like are used. However, even the use of these compounds has not exerted sufficient effects or has brought about the deterioration of the sharpness. Japanese Patent O.P.I. Publication No. 133734/1981 describes excellent magenta dye forming couplers which require no competing couplers. The use of such couplers, since the production of one molecule of the magenta dye requires 4 equivalents or 6 equivalents of silver, enables to restrain the developing agent from producing an excessive amount of the oxidized product thereof and the graininess from being deteriorated. However, those magenta dye forming couplers disclosed in the foregoing publication are very unstable; particularly unstable when left as they are, so that they cannot be put to practical use. And the densities of the magenta dyes formed from the above couplers are not sufficient, either.

It is therefore a first object of the present invention to provide a novel blocked magenta dye forming coupler intended to give a silver halide color photographic light-sensitive material having a high sensitivity and an excellent graininess.

It is a second object of the present invention to provide a novel blocked magenta dye forming coupler intended to give a

silver halide color photographic light-sensitive material having a high sensitivity and an excellent graininess without use of any competing couplers.

It is a third object of the present invention to provide a blocked magenta dye forming coupler which is excellent in the preservability under the unprocessed condition as well as in the color formability.

These objects of the present invention are accomplished by the use of a blocked magenta dye forming coupler (hereinafter referred to as the coupler of the present invention) having the formula:

Formula (I)

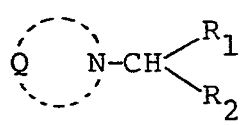

wherein Q represents a group of non-metallic atoms necessary to form a magenta dye forming coupler together with a nitrogen atom; $R_1$-CH-$R_2$ is a blocking radical which, after reacting with an oxidized developing agent, is released from the foregoing coupler of the present invention; $R_1$ is Z-NHCO-, Z-CO- or Z-O-CO- wherein Z is a 5- or 6-member heterocyclic ring which may be allowed to have a condensed ring and $R_2$ is X-NHCO-, X-CO-, X-O-CO- NC-, $O_2N$-, HOOC- (wherein X is an alkyl radical or an aryl radical) or any of the same radicals as defiend in $R_1$.

Examples of the heterocyclic ring represented by the Z

defined in $R_1$ include imidazole, oxazole, thiazole, thiophene, furanpyrrole, pyridine, pyrimidine, pyridazine, pyrazine, pyrazole, pyrrolidine, imidazoline, pyrazolidine, piperidine, piperazine, oxazoline, thiazoline, oxadiazole, thiadiazole, and the like, and further, examples of the condensed ring-having heterocyclic ring include quinoline, isoquinoline, indole, isoindole, benzothiazole, benzoxazole, carbazole, indoline, chroman, phenothiazine, quinazoline, naphthylidine, purine, and the like.

These heterocyclic rings also include those substituted by such a radical as an alkyl, an aryl, a halogen, an alkoxy, an aryloxy, carboxy, sulfo, cyano, nitro, an acyl, oxo, an alkoxycarbonyl, hydroxy, carbamoyl, sulfamoyl, amino, an acylamino, sulfamido, an alkyl-thio, or the like.

Radicals represented by the X defined in $R_2$ include alkyl radicals such as, e.g., methyl, ethyl, isopropyl, n-butyl, isobutyl, t-butyl, sec-amyl, n-octyl, sec-dodecyl, n-octadecyl, and the like, and aryl radicals such as, e.g., phenyl, naphthyl, and the like. These alkyl and aryl radicals also include those substituted by such a radical as an alkyl, an aryl, a halogen, an alkoxy, an aryloxy, carboxy, sulfo, cyano, nitro, an acyl, an alkoxycarbonyl, hydroxy, carbamoyl, sulfamoyl, amino, an acylamino, sulfonamido, an alkyl-thio, or the like.

Preferred magenta dye forming couplers formed with Q (hereinafter referred to as the magenta coupler of the present invention) are those conventionally known pyrazolo-triazole, pyrazolo-benzimidazole and indazolone compounds having the following formulas (II), (III) and (IV), respectively:

(II)

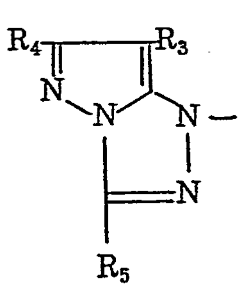

(III)

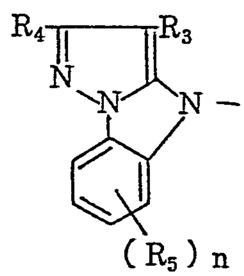

(IV)

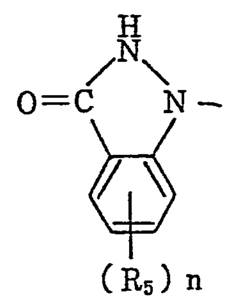

wherein $R_3$ represents hydrogen or a radical that is eliminatable during a color development, examples of which include a halogen atom, an alkoxy, an aryloxy, a heterocyclicoxy, sulfonyloxy, an acyloxy, a heterocyclic, thiocyano, an alkyl-thio, an aryl-thio, a heterocyclic thio, sulfonamido, phosphonyloxy, an arylazo, a substituted methyl (preferably alkyl or aryl substitute methyl), or a α-substituted benzyl radical (preferably α-alkyl or aryl substituted benzyl); $R_4$ and $R_5$ are independent of each other and each represents an alkyl, an aryl, a heterocyclic, an alkoxy, amino, an acylamino, anilino, hydroxy, an aryloxy, an alkyl-thio, an aryl-thio, carbamoyl, carboxy, an alkoxycarbonyl, ureido, imido, sulfonamido, sulfamoyl, or sulfo radical: To be more concrete, examples of the alkyl radical include, e.g., such straight-chained or branch-chained alkyl radicals having from 1 to 32 carbon atoms as methyl, isopropyl, t-butyl, dodecyl, and the like, and such cyclic alkyl radicals

as cyclopentyl, cyclohexyl, norbornyl, and the like, and these radicals are allowed to be further substituted by a halogen atom, nitro, cyano, an aryl, an alkoxy, an aryloxy, carboxy, an alkylthio, an aryl-thio, carbonyl, sulfamoyl, an acylamino, a heterocyclic radical, or the like. Examples of the aryl radical include such aryl radicals as phenyl, α-naphthyl, β-naphthyl, and the like, and these radicals are allowed to be further substituted by a halogen atom, an alkyl, nitro, cyano, an aryl, an alkoxy, an aryloxy, carboxy, sulfamoyl, carbamoyl, an acylamino, a diacylamino radical or the like.

Examples of the heterocyclic radical include 5-member of 6-member heterocyclic radicals containing such heteroatoms as nitrogen, oxygen and sulfur atoms (the heterocyclic radicals include those having a condensed ring) such as, e.g., pyridyl, quinolyl, furyl, benzothiazolyl, oxazolyl, imidazolyl, naphthoxazolyl, and the like, and these heterocyclic radicals are allowed to be further substituted by similar substituents to those in the above aryl radicals; and also include alkoxy radicals such as methoxy, ethoxy, and the like, alkylamino radicals such as, e.g., n-butylamino, dimethylamino, and the like, cycloamino radicals such as, e.g., piperidino, pyrrolidino, and the like, or such amino radicals as, e.g., heterocyclic amino radiclas, acylamino radicals such as, e.g., alkyl-acylamino radiclas, aryl-acylamino radicals, and the like, anilino radicals such as, e.g., 2,5-di-substituted anilino, and the like, ureido

radicals such as phenyl ureido, N,N-di-substituted ureido, and the like. And these radicals are allowed to be further substituted by similar substituents to those defined in the foregoing aryl radical.

Those preferred as the above-enumerated $R_4$ and $R_5$ are the aryl radicals typified by, e.g., phenyl, naphthyl, 2-chlorophenyl, 2,6-dichlorophenyl, 2,4,6-trichlorophenyl, 3,5-dibromophenyl, 3-nitrophenyl, 4-(2,4-di-t-amyl-phenoxy)acetamidophenyl, pentafluorophenyl, 4-phenoxyphenyl, 2,6-dimethyl-4-methoxyphenyl, 3-(N,N-diethylsulfamyl)phenyl, 2,6-dichloro-4-methoxyphenyl, 2-chloro-4,6-di-methyl-phenyl, pentachlorophenyl, 2,6-dichloro-4-carboxyphenyl, 2,5-dimethoxy-3,4-dichlorophenyl, 4-{α-(3-pentadecylphenoxy)butylamido}phenyl radicals and the like; the heterocyclic radicals typified by 2-thiazolyl, 2-benzothiazolyl, 2-benzoxazolyl, 2-imidazolyl, 2-benzoimidazolyl radicals and the like; the alkyl radicals typified by methyl, ethyl, dodecyl, 1-phenyl-trifluoroethyl radicals, and the like; the acylamino radicals typified by α-(3-pentadecyl-phenoxy)butylamido, n-tetradecaneamido, α-(2,4-di-t-amyl-phenoxy)butylamido, 3-[α-(2,4-di-t-amyl-phenoxy)butylamido]-benzamido, 3-acetylamidobenzamido radicals, and the like; the anilino radicals or phenyl ureido radicals typified by phenylamino, 2-chlorophenylamino, 2,4-dichlorophenylamino, 2,4-dichloro-5-hexadecyloxyanilino, 2-chloro-5-tetradecaneamidoanilino, 2-chloro-5-octadesenylsuccinimidoanilino, 2-chloro-5-[α-{3-t-butyl-4-hydroxy)phenoxy}

tetradecaneamido]anilino, 2-chloro-3-[2-{1,3-(1-hexadecyl-5-trifluoromethyl)benzimidazo}lyl]anilino, 2-methoxy-5-(4-hexadecyloxybenzamido)anilino radicals, and the like; and the ureido radicals typified by 3-{α-(2,4-di-t-amyl-phenoxy)butylamido} phenylureido radicals and the like. n is preferably from 1 to 4.

The following are exemplified compounds of the magenta coupler of the present invention, but the present invention is not limited thereto.

(1)

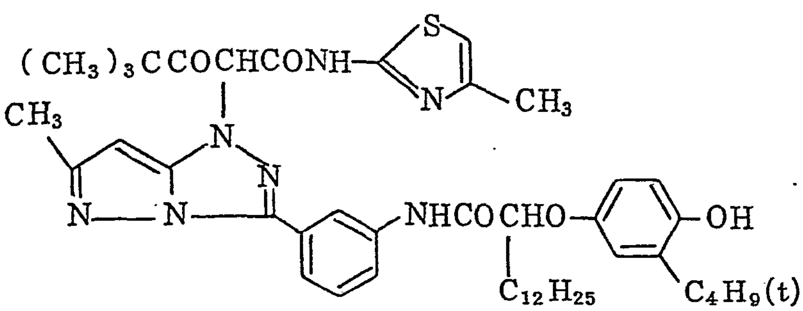

(2)

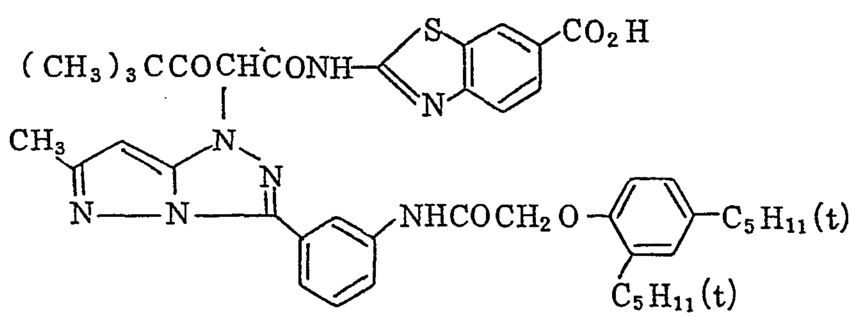

(3)

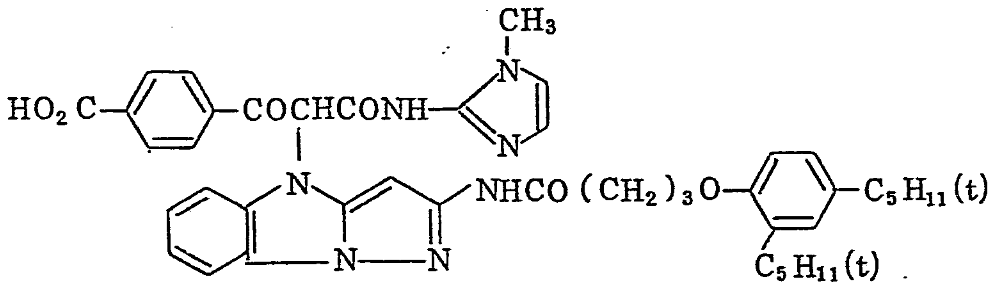

(4)

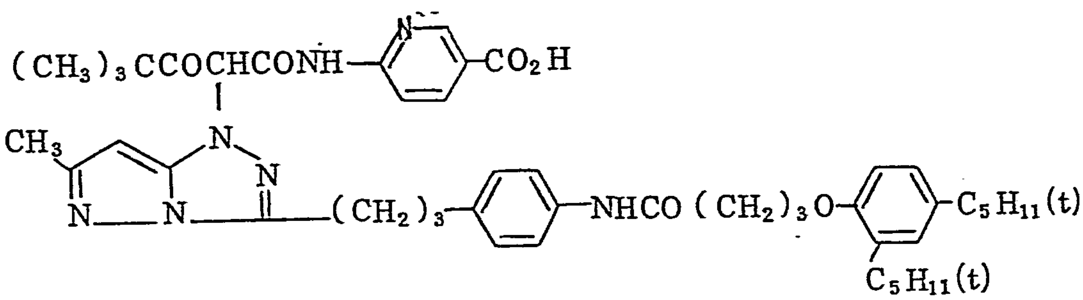

(5)

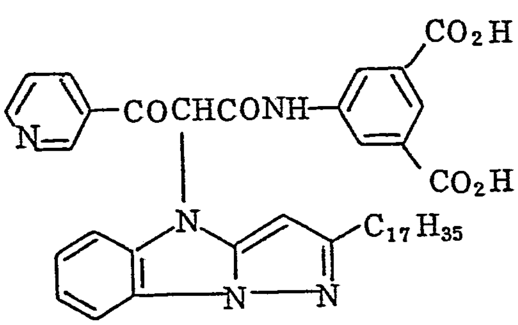

(6)

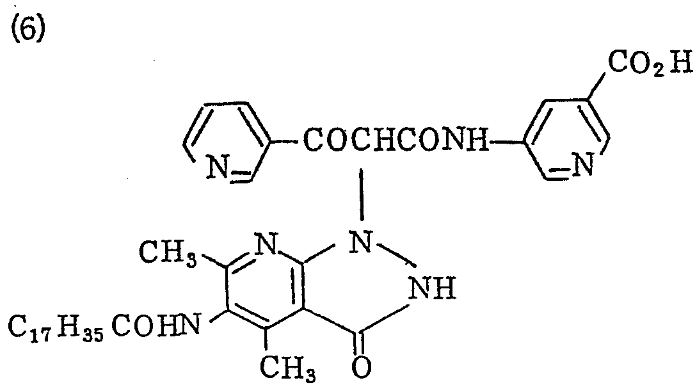

(7)

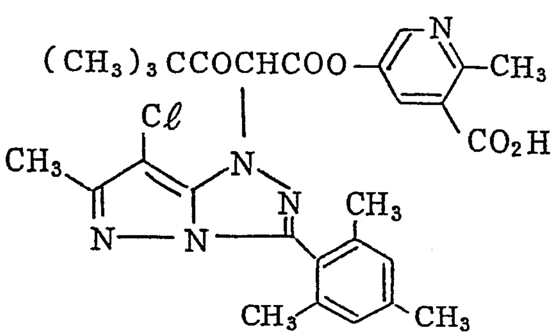

(8)

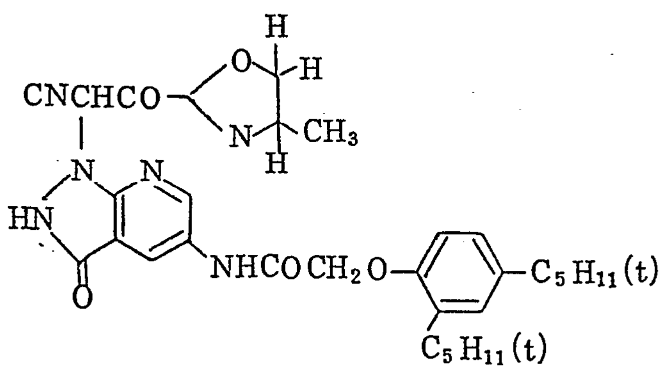

(9)

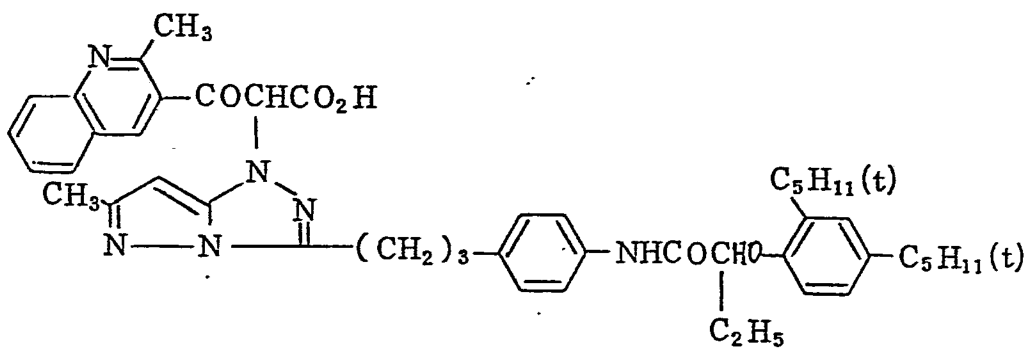

(10)

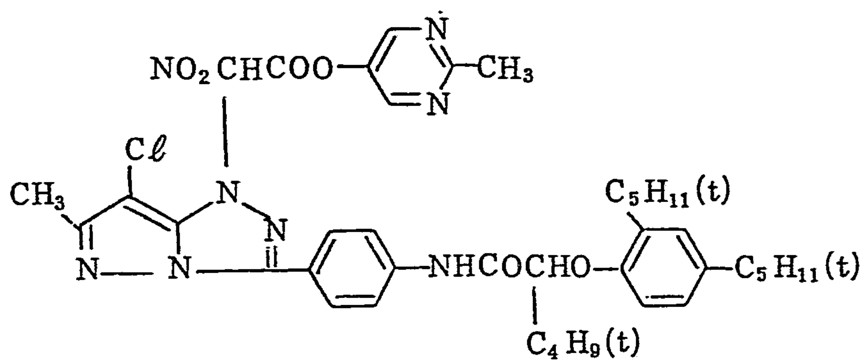

(11)

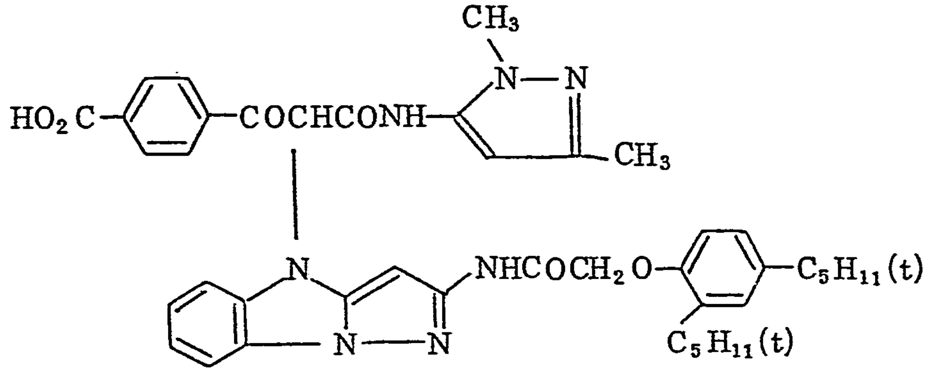

(12)

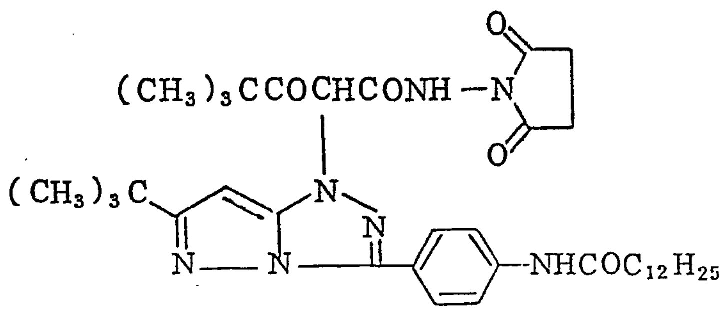

(13)

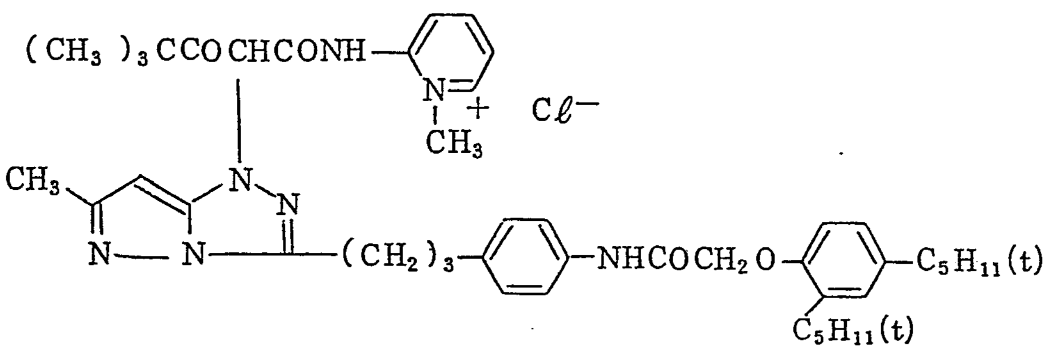

(14)

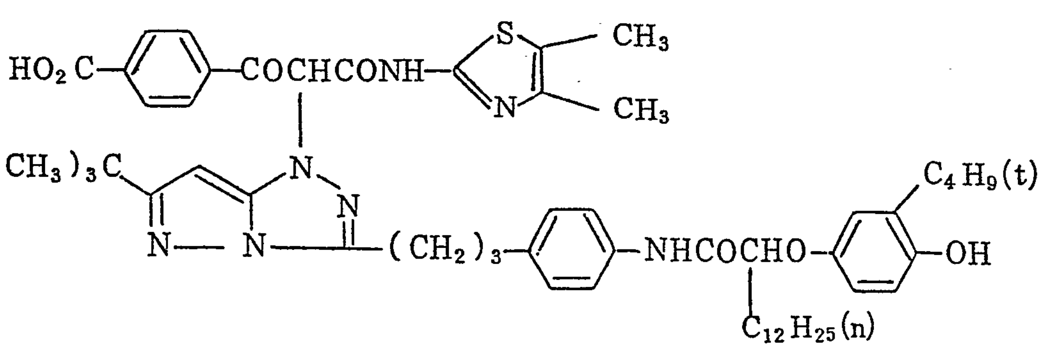

(15)

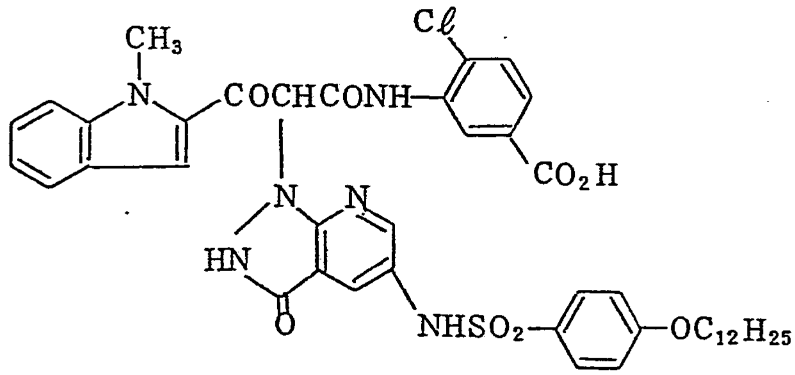

(16)

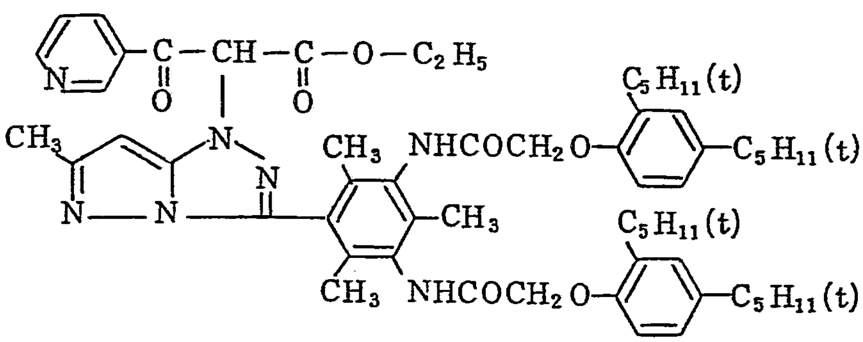

(17)

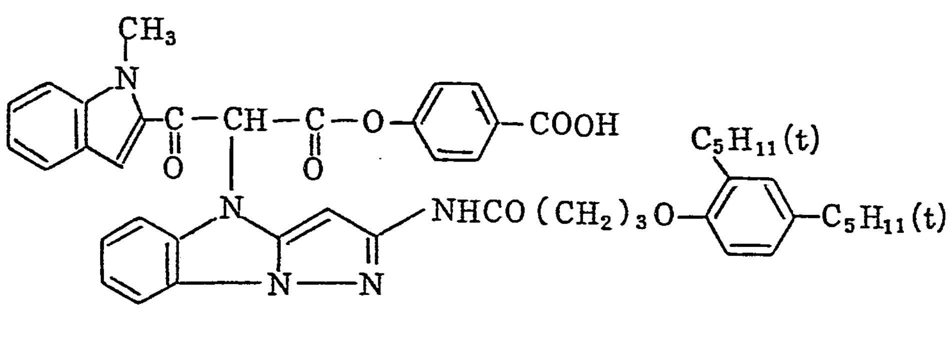

(18)

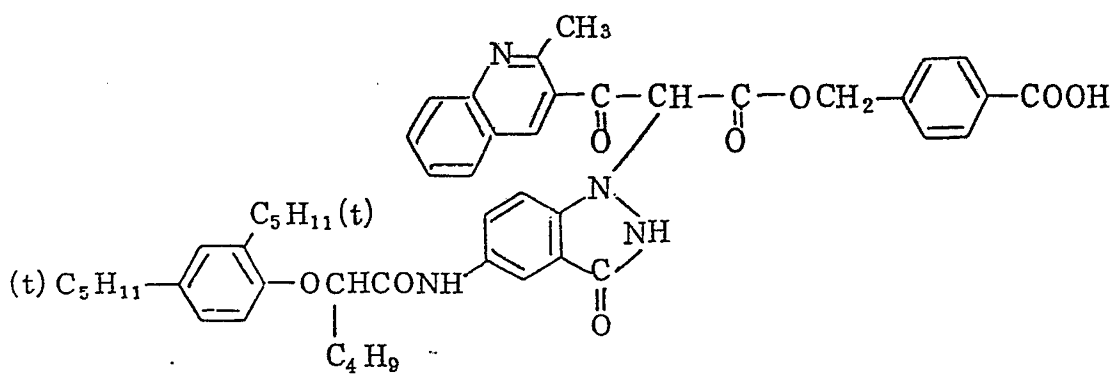

The present invention is to provide a magenta dye forming coupler having a high equivalency and excellent color formability. The meaning of the "having a high equivalency" is as has been previously mentioned, but the necessity of the rapid color formability is concerned with the graininess because the presence of an excessive amount of a developing agent brings about the bleach of the developing silver nucleus.

It has been found that the coupler of the present invention consists of the part that couples with oxidized developing agent to thereby form a magenta dye and the part that does not, and of these parts the latter takes part in the preservability as well as in the color formability of the coupler.

The coupler of the present invention can be synthesized in the presence of a base by the condensation reaction of a compound having the formula:

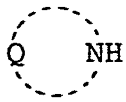

with that having the formula:

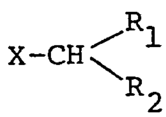

wherein X is a halogen atom (e.g., chlorine or bromine), Q, $R_1$ and $R_2$ are as defined in Formula (I).

Usable solvents include alcohol, ethyl acetate, chloroform, toluene, dimethyl formamide, dimethyl sulfoxide, and the like, and usable bases include potassium hydroxide, sodium hydroxide, potassium carbonate, triethylamine, pyridine, sodium methoxide, and the like. the 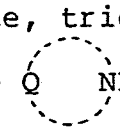 may be used in such the salt form as 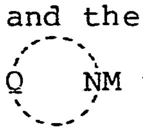 wherein M is calcium, sodium, potassium, or the like.

The following is an example of the synthesis of a coupler of the present invention, but the present invention is not limited thereto.

Synthesis Example

(Synthesis of Exemplified coupler 2)

A synthesis was carried out in accordance with the following synthesis route:

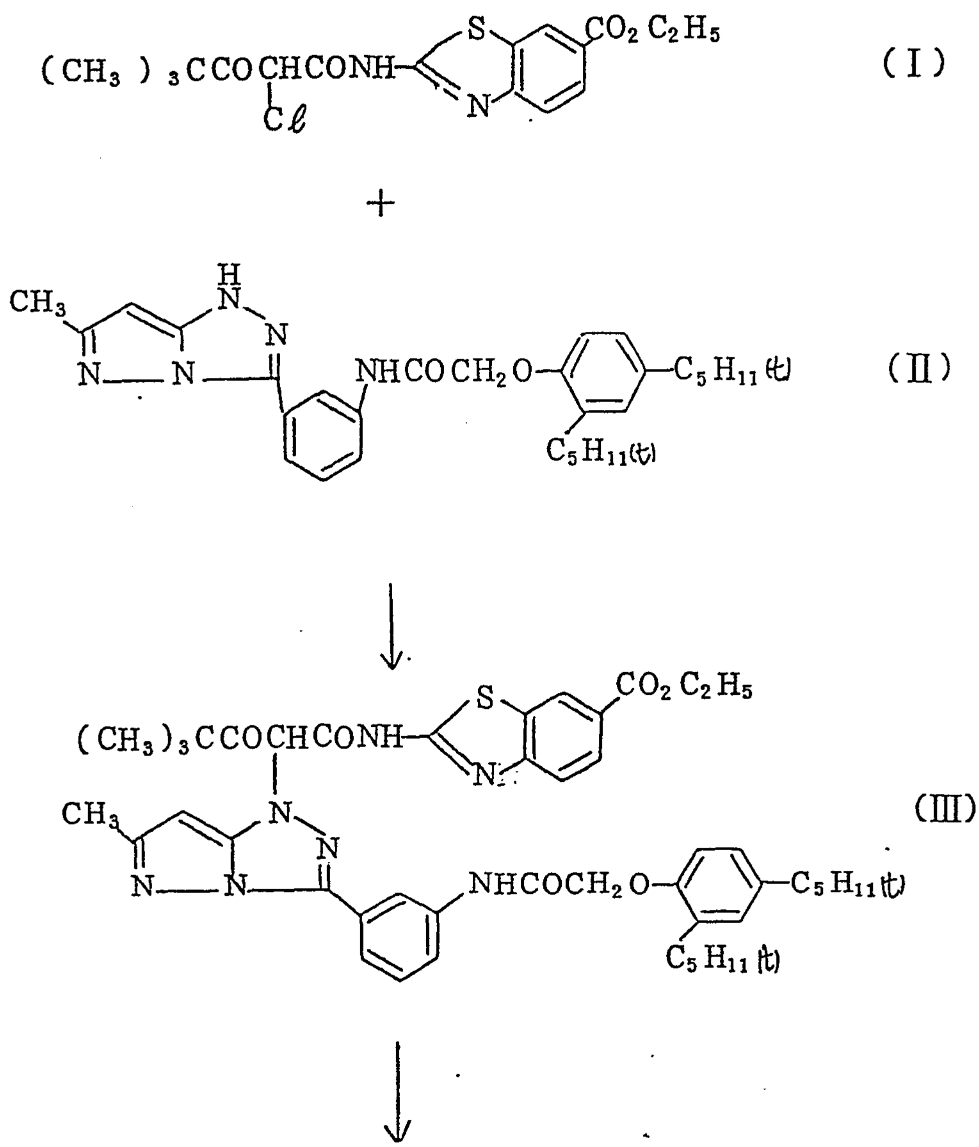

(I)

(II)

(III)

Exemplified Coupler 2

4.9 g of compound (II) was dissolved into 20 ml of anhydrous DMF, and to the solution, with cooling by ice, were added 0.5 g of sodium hydride (50%). After completion of the generation of a hydrogen gas, 3.9 g of compound (I) were added to the mixture, and the mixture was stirred over a period of four hours at room

temperature. The reaction liquid was poured into a hydrochloric-acidified ice, and the produced solids were collected and dried. The solid was dissolved into a small amount of benzene to isolate an objective product (IV) therefrom by the silica gel column chromatography that uses benzene-ethyl acetate solvent as the development solvent thereof, whereby about 6 g of compound (III) were obtained. 5 g of the thus obtained compound (III) was dissolved into 50 ml of alcohol, to which was added an aqueous 10% potassium hydroxide solution and was stirred for three hours at room temperature. The reaction liquid was poured into hydrochloric-acidified ice, and the produced solids were collected and then dried. The resulting solid was recrystallized by use of acetonitrile, whereby 3.2 g of Exemplified Coupler 2 were obtained. m.p. 145 to 148°C NMR, ascertained by the procedure of mass spectrometry.

Other exemplified compounds may also be synthesized in like manner.

Those couplers of the present invention produced by the above-described method may be put to photographic use to form an excellent color image. For use, those soluble in an aqueous alkaline solution may be used as the so-called coupler-in-developer type coupler to be added to a developer liquid, while those non-alkali-soluble may be used as the so-called coupler-in-emulsion type coupler to be dissolved into such a high boiling solvent as dioctyl-butyl phosphate, tributyl phosphate,

tricresyl phosphate, dibutyl phthalate, diethyl-laurylamide, 1,4-dicyclohexyl-methyl-2-ethyl hexanoate, or the like, and/or such a low boiling solvent as ethyl acetate, methanol, acetone, tetrahydrofuran, or the like, and then added to a component layer of a light-sensitive material.

And the photographic light-sensitive material into which is incorporated the coupler of the present invention is composed of at least a support and light-sensitive layers provided thereon, but the composition, according to purposes, may be of various component layers generally constituted by the light-sensitive layers thereof and/or other component layers thereof such as interlayers, subbing layer, filter layers, protective layer, image receiving layers, and the like.

As the light-sensitive layer, a red, a green, a blue-sensitive layers are provided generally. And the layers which have same color-sensitivity may comprise a higher-speed emulsion layer and a lower-speed emulsion layer. When adding to the light-sensitive layers, the coupler of the present invention may be added to any component layer of the emulsion, but is desirable to be added to the green-sensitive emulsion layer and/or to the higher-speed emulsion layer and/or more preferably to the higher-speed emulsion layer of green-sensitive emulsion layers. And adding quantity thereof should be from 0.01 to 3.0 moles, and preferably from 0.1 to 1.0 mole per mole of the silver halide used in the layer.

The above silver halide used in the present invention may be an arbitrary silver halide such as silver chloride, silver iodide, silver iodobromide, silver chlorobromide, silver chloroiodobromide, or the like, and these silver halides may be produced by various processes such as the neutral process, the ammonia process, and the like. And these silver halides may be chemically sensitized by the single or arbitrarily combined use of active gelatin, sulfur sensitizers such as, e.g., aryl-thiocarbamide, thiourea, cystine, and the like, selenium sensitizers, reduction sensitizers such as, e.g., stannous salts, polyamines, and the like, noble-metallic sensitizers such as, e.g., watersoluble salts of gold, ruthenium, rhodium, indium, and the like.

Further, these emulsions may be optically sensitized to desired wavelength regions by the single or arbitrarily combined use of optical sensitizers including such cyanine dyes as, e.g., zeromethine dyes, monomethine dyes, dimethine dyes, trimethine dyes, and merocyanine dyes, and the like.

As materials for the support of the light-sensitive material, known materials in the film or sheet form may be used which include paper, laminated paper, glass, cellulose acetate, polyester, polycarbonate, and the like.

Couplers of the present invention may be used in combination thereof, and may also be used in combination with other two-equivalent or four-equivalent couplers or with colored couplers, and the like, and appropriate couplers selected from

the respective couplers differing in the color of the dye to be produced therefrom are to be incorporated into corresponding light-sensitive layers that are sensitive to appropriate wavelength regions respectively.

Namely, in the production of a multi-layer color photographic light-sensitive material of the present invention, as the couplers differing in the color of the dye to be formed therefrom, for example, benzoyl acetanilide or pivaloyl acetanilide yellow couplers, phenol or naphthol cyan couplers, DIR couplers, colored couplers or other different magenta couplers, and the like may at need be selectively used. These couplers are described in, e.g., Japanese Patent O.P.I. Publication Nos. 29432/1973, 66834/1973, 112038/1975, 52423/1978, 109630/1978, 133329/1979 and 145135/1979, Japanese Patent Examined Publication No. 37854/1974, U.S. Patent No. 3,684,514, and the like, and also in Research Disclosure Nos. 19633 and 19536, and the like, and from these couplers described in the publications appropriate ones may be selected to be used for the foregoing purpose.

The photographic light-sensitive material may, according to purposes, contain various photographic additives in the light-sensitive layers thereof and/or other component layers thereof such as interlayers, subbing layer, filter layers, protective layer, image receiving layers, and the like, which photographic additives include, e.g., stabilizers, sensitizers, layer's

physical property improving agents, hardeners, coating aids, coupler solvents, the so-called DIR compounds that release development inhibitors during color development and produce substantially colorless compounds, and as others, antistatic agents, defoaming agents, ultraviolet absorbing agents, brightening agents, antislipping agents, matting agents, antihalation or antiiradiation agents, and the like. These various additives may be used singly or in combination.

On the other hand, the color developer to be used in color-developing an exposed color photographic light-sensitive material consists principally of a color developing agent as previously mentioned, but the color developing agent to be used in the present invention is an aromatic primary amine typified by p-phenylenediamines, particularly useful ones of which are 3-methyl-4-amino-N-ethyl-N-β-hydroxyethyl-aniline, 3-methyl-4-amino-N-ethyl-N-β-methanesulfonamidoethyl-aniline, 3-methyl-4-amino-N-ethyl-N-β-methoxyethyl-aniline, and the like.

After color development, in order to remove the silver halide or the developed silver in the light-sensitive material therefrom to the outside of the system, generally a bleach-fixer liquid is used. As the fixer component of the liquid, such a silver halide solvent as sodium thiosulfate, ammonium thiosulfate, or the like is used, while as the bleaching component of the liquid, red prussiate potash, ammonium salt or sodium salt of ferric ethylenediamine-tetraacetic acid, or the

like is used. The light-sensitive material containing the coupler of the present invention may also be processed in an aqueous alkaline solution, the so-called alkaline activator.

The silver halide color photographic light-sensitive material containing the coupler of the present invention may be used as, e.g., a diffusion transfer type silver halide light-sensitive material, a negative light-sensitive material for general use, a reversal light-sensitive material for general use, a positive light-sensitive material for general use, a direct-positive type light-sensitive material, or the like.

In addition to the above, the coupler of the present invention may also be applied to a conventional light-sensitive material of which the quantity of the silver halide is extremely reduced and which is subjected to an amplifying treatment that uses a cobalt (IV) complex or hydrogen peroxide.

As has been described in detail, the coupler of the present invention having the structure represented by the foregoing general formula is excellent in such photographic characteristics as the sensitivity, maximum density, fog, yellow stain, and the like, and further improved on the stability during the storage thereof, so that the coupler can be used extensively effectively, particularly, in the silver-saving color photographic technology.

The present invention is illustrated further in detail with reference to examples below, but the embodiments of the

present invention are not limited thereto.

Example 1

Exemplified Coupler 1 in the quantity of $2x10^{-2}$ mole was taken to be dissolved into a mixture of tricresyl phosphate in the same quantity as that of the coupler with ethyl acetate in the quantity of three times that of the coupler, and after that, the solution was mixed with 300 ml of a 5% aqueous gelatin solution containing 1.5 g of Alkanol B (alkyl-naphthalene sulfonate, manufactured by DuPont); and then emulsified to be dispersed by means of a colloid mill. The dispersed liquid of this coupler was then mixed with 1 kg of a photographic emulsion containing $8x10^{-2}$ mole of green-sensitive silver iodobromide (6 mole% of silver iodide and 94 mole% of silver bromide) and 40% of gelatin, to which was then added as a hardener 20 ml of 2% aqueous solution of 1,2-bis(vinyl-sulfonyl)-ethane, and the resulting liquid was coated on a triacetate film base to thereby prepare a color light-sensitive material sample 1. The coated quantity of silver used in the preparation of sample 1 was $2g/m^2$.

Further, in place of the Exemplified Coupler 1, exemplified Couplers 4 and 13 were used to prepare samples 2 and 3 in quite the same manner as in the above.

On the other hand, for comparison, in place of the Exemplified Coupler, control couplers A and B were used to prepare samples 4 and 5 in quite the same manner as in the above.

Control Coupler A

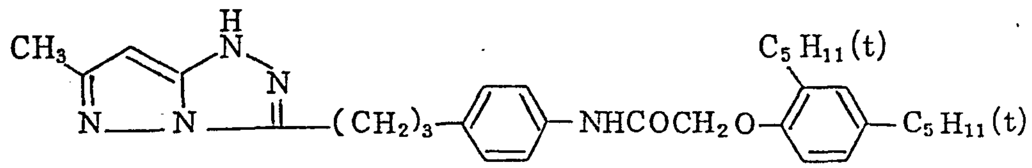

Control Coupler B

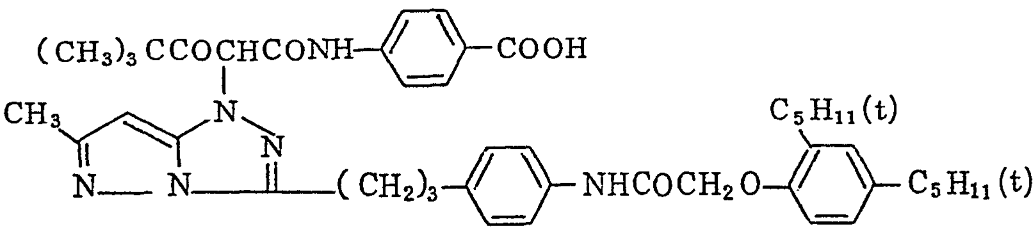

These samples 1, 2, 3, 4 and 5 were exposed through an optical wedge to light in the ordinary manner, and then processed in the following steps with use of the processing compositions given below.

On the other hand, other two groups of samples prepared by subjecting samples 1 to 5, unexposed to light, to accelerating aging treatments of 80%RH at 50°C for three days and of 10%RH at 55°C for three days were also exposed through an optical wedge to light and processed in the same manner as in the above-mentioned samples.

Processing steps (at 38°C):

| | |
|---|---|
| Color development | 3 minutes and 15 seconds |
| Bleaching | 6 minutes and 30 seconds |
| Washing | 3 minutes and 15 seconds |

| | |
|---|---|
| Fixing | 6 minutes and 30 seconds |
| Washing | 3 minutes and 15 seconds |
| Stabilizing | 1 minute and 30 seconds |

Color developer:

| | |
|---|---|
| 4-amino-3-methyl-N-ehtyl-N-(β-hydroxyethyl)-aniline sulfate | 4.75 g |
| Anhydrous sodium sulfite | 4.25 g |
| Hydroxylamine 1/2 sulfate | 2.0 g |
| Anhydrous potassium carbonate | 37.5 g |
| Sodium bromide | 1.3 g |
| Trisodium nitrilotriacetate, monohydrated | 2.5 g |
| Potassium hydroxide | 1.0 g |
| Water to make 1 liter | |

Potassium hydroxide is used to adjust the pH to 10.0

Bleaching bath:

| | |
|---|---|
| Ammonium ethylenediamine-tetraacetate | 100.0 g |
| Diammonium ethylenediamine-tetraacetate | 10.0 g |
| Ammonium bromide | 150.0 g |
| Glacial acetic acid | 10.0 ml |
| Water to make 1 liter | |

Aqueous ammonia is used to adjust the pH to 6.0

Fixing bath:

| | |
|---|---|
| Ammonium thiosulfate (50% aqueous solution) | 162 ml |

| | |
|---|---|
| Anhydrous sodium sulfite | 12.4 g |
| Water to make 1 liter | |

Acetic acid is used to adjsut the pH to 6.5

Stabilizing bath:

| | |
|---|---|
| Formalin (37% aqueous solution) | 5.0 ml |
| Koniducks (manufactured by Konishiroku Photo Industry Co., Ltd.) | 7.5 ml |
| Water to make 1 liter | |

The magenta dye images obtained by processing in the above-mentioned manner were measured with use of a green light by means of a densitometer PD-7R (manufactured by Konishiroku Photo Industry Co., Ltd.). The results are as shown in Table 1 wherein the sensitivities of the non-aging samples are shown with the relative speed values to the value of sample 5 which is regarded as 100, while the sensitivities of the aging samples subjected to 80%RH/50°C and 10%RH/55°C treatments are shown each with the relative speed to the non-aging speed value regarded as 100 of each of the samples. As to the fog and Dmax, the results are shown with actual values in Table 1.

Table 1

| Sample No. | Coupler | Non-aging | | | Aging | | | | | |
|---|---|---|---|---|---|---|---|---|---|---|
| | | | | | 80%RH/50°C | | | 10%RH/55°C | | |
| | | Sensitivity | Fog | Dm | Sensitivity | Fog | Dm | Sensitivity | Fog | Dm |
| 1 | Exemplified coupler 1 | 109 | 0.12 | 1.59 | 89 | 0.12 | 1.55 | 89 | 0.12 | 1.55 |
| 2 | Exemplified coupler 4 | 112 | 0.13 | 1.58 | 89 | 0.13 | 1.55 | 90 | 0.13 | 1.54 |
| 3 | Exemplified coupler 13 | 115 | 0.16 | 1.63 | 85 | 0.18 | 1.58 | 86 | 0.18 | 1.57 |
| 4 | Control coupler A | 65 | 0.20 | 0.70 | 80 | 0.22 | 0.63 | 83 | 0.25 | 0.60 |
| 5 | Control coupler B | 100 | 0.12 | 1.56 | 35 | 0.15 | 0.58 | 32 | 0.18 | 0.58 |

As seen from Table 1, samples 1, 2 and 3 of the present invention show as satisfactory results as having obviously higher sensitivities, higher Dmax, and less fog than those of the samples in which control couplers A and B were used. And even in the endurance test (preservability) against the 80%RH/50°C and 10%RH/55°C treatments, sample 5 wherein control couplers B was used shows that the preservability thereof is so poor as to produce unsatisfactory color formation, whereas the samples wherein the couplers of the present invention were used show as good results as to be very stable against heat as well as against moisture.

On the other hand, the densities of the above processed non-aging samples 1 to 5 were measured through a green filter, and then measured on the same portion through an yellow filter to thereby examine the turbidity of the color (DE/DGx100) formed on the samples. The examined results are given in Table 2.

Table 2

| Sample No. | Color turbidity |
|---|---|
| 1 | 25 |
| 2 | 19 |
| 3 | 18 |
| 4 (control) | 18 |
| 5 (control) | 27 |

As apparent from Table 2, all the samples show nearly the same color turbidity, and therefore the samples of the invention are much comparable to the sample containing control coupler A that has no blocking radical, thus proving that the blocking radical has flowed out of the system.

Example 2

Exemplified Coupler 3 was used to prepare sample 6 in quite the same procedure as in Example 1.

Further, in place of the Exemplified Coupler 3, Exemplified

Couplers 5 and 11 were used to prepare samples 7 and 8 also in quite the same procedure as in Example 1.

On the other hand, in place of the Exemplified Coupler 3, control couplers B and C were used to prepare samples 9 and 10 also in quite the same procedure.

Further, for comparison, $2x10^{-2}$ mole of control coupler C was used together with $1x10^{-3}$ mole of DIR-R to prepare sample 11 also in quite the same procedure.

Control Coupler C

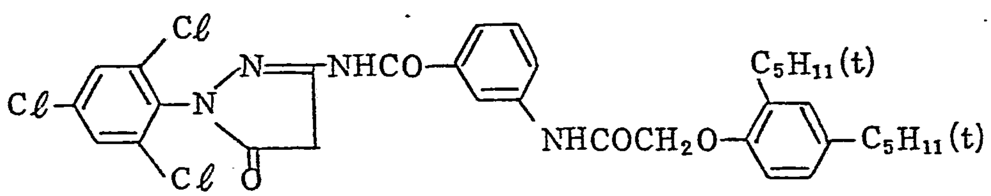

DIR-D

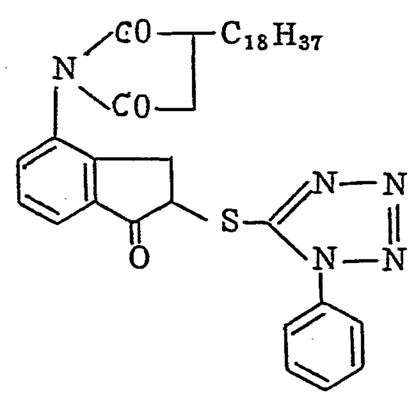

These samples 6, 7, 8, 9, 10 and 11 were exposed and processed in the same manner as in Example 1, and the photographic characteristics of the samples were examined.

In addition, these processed samples were measured with

use of a green light for the graininess of the respective dyes formed thereon at the density of 0.7 by the RMS (Root-Mean Square) method. The measured results are as given in Table 3.

Table 3

| Sample No. | Coupler | Color developing characteristics | | | | |
|---|---|---|---|---|---|---|
| | | Sensi-tivity | Fog | Gamma | Dm | RMS value |
| 6 | Exemplified coupler 3 | 90 | 0.12 | 0.61 | 1.62 | 54 |
| 7 | Exemplified coupler 5 | 88 | 0.11 | 0.67 | 1.60 | 55 |
| 8 | Exemplified coupler 11 | 89 | 0.12 | 0.65 | 1.61 | 54 |
| 9 | Control coupler B | 65 | 0.12 | 0.62 | 1.56 | 55 |
| 10 | Control coupler C | 100 | 0.13 | 1.30 | 2.50 | 70 |
| 11 | Control coupler C + DIR-D | 75 | 0.11 | 0.73 | 1.61 | 50 |

In addition, in the above table, the sensitivities are shown with the relative speeds to the speed regarded as 100 of sample 10.

As apparent from Table 3, the samples to which the couplers of the present invention were applied are obviously improved on the graininess to be better than the sample containing control coupler C, and show about the same results in the graininess as

those of sample 11 containing control coupler C plus DIR compound and of sample 9 containing control coupler B.

On the other hand, in a sealed container containing 1% aqueous formaldehyde solution in the darkroom unexposed samples 6 to 11 were allowed to stand over a period of three days, the samples being arranged inside the container so as not to touch the solution. These samples and another group of samples not subjected to the above treatment prepared for comparison were exposed and processed in the same manner as in Example 1, and then measured for the sensitivities and maximum densities thereof to thereby find the endurance (%) thereof against formalin (treated sample/untreated sample x 100). The obtained results are as shown in Table 4.

Further, unexposed samples 6 to 11 were stored for three days under the condition of 80%RH/50°C. These treated samples and control samples treated likewise for comparison were exposed and processed in the same manner as in Example 1, and then measured for the maximum densities thereof to thereby examine the preservability thereof. The examined results are as given in Table 4. In addition, the values of the preservability are what has been calculated according to the formula: treated sample/untreated sample x 100.

Table 4

| Sample No. | Endurance against formalin (%) | | 80%RH/50°C |
|---|---|---|---|
| | Sensitivity | Dmax | Preservability (%) |
| 6 | 94 | 98 | 89 |
| 7 | 93 | 95 | 87 |
| 8 | 92 | 96 | 88 |
| 9 | 90 | 88 | 37 |
| 10 | 73 | 66 | 85 |
| 11 | 70 | 64 | 70 |

From Table 4, it is understood that the samples in which the couplers of the invention were used are apparently excellent in the endurance against formalin as compared to the sample containing control coupler C.

As to the preservability, the samples containing the couplers of the invention are all satisfactory; particularly a conspicuous difference in the effect is observed between the samples of the invention and the sample containing control coupler B.

Example 3

Exemplified Coupler 2 in the quantity of $1 \times 10^{-2}$ mole was taken to be dissolved into a mixture of dibutyl phthalate in

the same quantity as that of the coupler with ethyl acetate in three times as much quantity as that of the coupler, and, after that, the solution was mixed with 150 ml of a 5% aqueous gelatin solution containing 1.5 g of Alkanol B (alkylene-naphthalene sulfonate, manufactured by DuPont), and subsequently emulsified to be dispersed by means of a colloid mill. The resulting coupler-dispersed liquid was mixed with 200 g of a photographic emulsion containing $5x10^{-2}$ mole of green-sensitive silver chlorobromide (20 mole% of silver chloride and 80 mole% of silver bromide) and 10 g of gelatin, and to the mixture were added as a hardener 10 ml of a 2% aqueous solution of 1,2-bis (vinyl-sulfonyl)ethane, and the resulting mixture was coated on a polyethylene-coated support and then dried, whereby a color light-sensitive material sample 12 was produced. The coated quantity of silver of sample 12 was 0.5 $g/m^2$.

In place of the Exemplified Coupler 2, Exemplified Coupler 14 was used to prepare sample 13 in quite the same manner as in the above.

On the other hand, in place of the Exemplified Coupler 2, control coupler B was used to prepare sample 14 also in quite the same manner as in the above.

These samples 12, 13 and 14 were exposed through an optical wedge to light in usual manner, and subsequently processed in the following processing steps with use of liquids having the compositions given below:

Processing steps (at 33°C):

| | |
|---|---|
| Color development | 3 minutes and 30 seconds |
| Bleach-fixing | 1 minute and 30 seconds |
| Washing | 3 minutes |

Color developer:

| | |
|---|---|
| 4-amino-3-methyl-N-ethyl-N-(β-methanesulfonamidoethyl)-aniline sulfate | 4.0 g |
| Benzyl alcohol | 10.0 ml |
| Hydroxylamine sulfate | 2.0 g |
| Potassium carbonate | 25.0 g |
| Potassium bromide | 0.2 g |
| Anhydrous sodium sulfite | 2.0 g |
| Diethylene glycol | 3.0 ml |
| Water to make 1 liter, adjust the pH to 10.0 | |

Bleach-fixing bath:

| | |
|---|---|
| Iron-sodium ethylene-tetraacetate | 60.0 g |
| Ammonium thiosulfate | 100.0 g |
| Sodium hydrogensulfite | 10.0 g |
| Sodium metabisulfite | 3.0 g |
| Water to make 1 liter, adjust the pH to 6.6 | |

The thus obtained samples 12, 13 and 14 were illuminated in a xenon fadometer over a period of 4 days to examine the respective image resistances to light and the yellow stain in the non-color-developed area. The examined results are as shown in Table 5. In addition, the light-resistant dye residual rates shown in the table are the values obtained at the initial density of 1.0.

Table 5

| Sample No. | Coupler | Resistance to light | |
|---|---|---|---|
| | | Dye residual rate | Yellow stain |
| 12 | Exemplified Coupler 2 | 79 | 0.13 |
| 13 | Exemplified Coupler 14 | 81 | 0.14 |
| 14 | Control Coupler B | 63 | 0.20 |

From Table 5, it is understood that the samples containing the couplers of the present invention has less dye residual rate and less yellow stain than those of sample 14 containing control cupler B.

CLAIMS

1. A blocked magenta dye forming coupler having the formula:

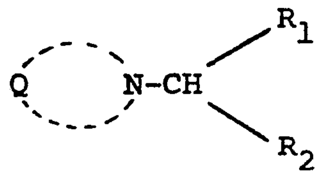

wherein Q represents a group of non-metallic atoms necessary to form a magenta dye forming coupler together with a nitrogen atom; and $R_1$-CH-$R_2$ represents a blocking radical which, after reacting with an oxidized developing agent, is released from said blocked magenta dye forming coupler where $R_1$ represents Z-NHCO-, Z-CO- or Z-O-CO- wherein Z is a 5- or 6-membered heterocyclic ring, which may have a condensed ring; and $R_2$ represents X-NHCO-, X-CO-, X-O-CO-, NC-, $O_2N$-, HOOC- (wherein X is an alkyl or an aryl radical) or a radical as defined in $R_1$.

2. A blocked magenta dye forming coupler according to claim 1, wherein Q 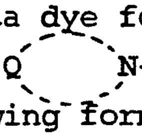 N- represents a group having one of the following formulae:

Formula (II)

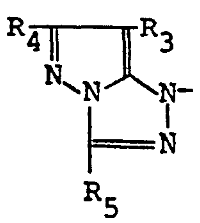

Formula (III)

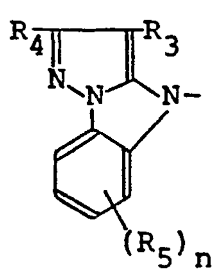

Formula (IV)

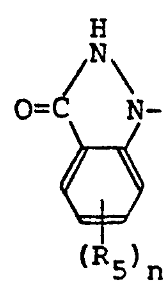

wherein $R_3$ represents a hydrogen atom or an eliminatable group capable of being released from the blocked magenta coupler upon reaction with an oxidized developing agent; $R_4$ and $R_5$ represent, independently, an alkyl, an aryl, a heterocyclic, an alkoxy, an amino, an acylamino, an arylamino, a hydroxy, an aryloxy, an alkyl-thio, an aryl-thio, a carbamoyl, a carboxy, an alkoxycarbonyl, a ureido, an imido, a sulfonamido, a sulfamoyl, or a sulfo radical; n represents an integer of 1 to 4.

3. A blocked magenta dye forming coupler according to claim 2, wherein the eliminatable group represented by $R_3$ is a halogen atom, an alkoxy, an aryloxy, a heterocyclic oxy, a sulfonyloxy, an acyloxy, a heterocyclic, a thiocyano, an alkyl-thio, an aryl-thio, a heterocyclic thio, a sulfonamido, a phosphonyloxy, an arylazo, a substituted methyl, or an α-substituted benzyl.

4. A blocked magenta dye forming coupler according to claim 1 having the formula:

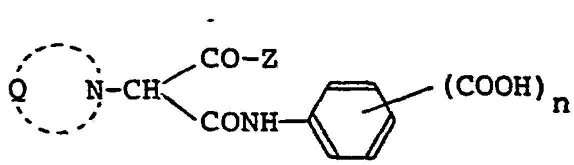

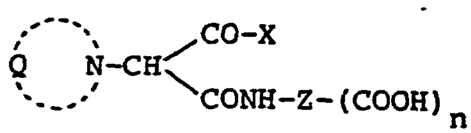

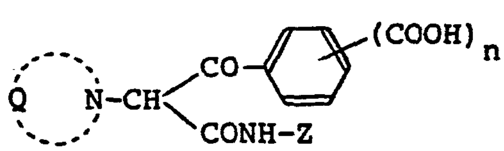

wherein Q, Z and X are as defined in claim 1 and n represents an integer of 1 or 2.

5. A blocked magenta dye forming coupler according to claim 1, 2, 3 or 4 wherein the heterocyclic ring Z is an imidazole, oxazole, thiazole, thiophene, furanpyrrole, pyridine, pyrimidine, pyridazine, pyrazine, pyrazole, pyrrolidine, imidazoline, pyrazolidine, piperidine, piperazine, oxazoline, thiazoline, oxadiazole, thiadiazole, quinoline, isoquinoline, indole, isoindole, benzothiazole, benzoxazole, carbazole, indoline, chroman, phenothiazine, quinazoline, naphthylidine or purine ring.

6. A silver halide photographic light-sensitive material comprising a support provided thereon with a silver halide emulsion layer comprising a coupler as claimed in any one of claims 1 to 5.

7. A silver halide photographic light-sensitive material according to claim 6 wherein the silver halide emulsion layer is a green-sensitive emulsion layer.

8. A silver halide photographic light-sensitive material according to claim 7 wherein the green-sensitive emulsion layer comprises a higher-speed emulsion layer and a lower-speed emulsion layer.

9. A silver halide photographic light-sensitive material according to claim 8 wherein the higher-speed emulsion layer comprises the said coupler.

10. A blocked magenta dye forming coupler according to claim 1 which has the formula (1) to (18).